Europäisches Patentamt

European Patent Office

Office européen des brevets

(11) Publication number: **0 182 412**
**B1**

## (12) EUROPEAN PATENT SPECIFICATION

(45) Date of publication of patent specification: **31.05.89**

(21) Application number: **85201717.7**

(22) Date of filing: **18.10.85**

(51) Int. Cl.⁴: **A 61 F 2/80**

(54) **An orthopaedic covering material and an artificial limb socket made from said material.**

(30) Priority: **19.10.84 NL 8403191**

(43) Date of publication of application:
**28.05.86 Bulletin 86/22**

(45) Publication of the grant of the patent:
**31.05.89 Bulletin 89/22**

(84) Designated Contracting States:
**BE DE FR GB NL**

(56) References cited:
**EP-A-0 045 592**
**FR-A-1 554 413**
**US-A-1 528 257**
**US-A-1 861 311**
**US-A-2 578 019**
**US-A-2 689 351**
**US-A-3 600 717**
**US-A-4 139 002**

(73) Proprietor: **Jongenengel, Cornelis Dirk**
**Baarsweg 1**
**NL-3192 VA Hoogvliet (NL)**

(72) Inventor: **Jongenengel, Cornelis Dirk**
**Baarsweg 1**
**NL-3192 VA Hoogvliet (NL)**

(74) Representative: **Smulders, Theodorus A.H.J., Ir.**
**et al**
**Vereenigde Octrooibureaux Nieuwe Parklaan**
**107**
**NL-2587 BP 's-Gravenhage (NL)**

## Description

This invention relates to an orthopaedic covering material made from a thin, flexible leather inner layer and a layer of resilient material attached to the exterior of said inner layer.

This invention relates more in particular to an artificial limb socket made from such orthopaedic covering material.

Orthopaedic covering material, which often abuts, under pressure, against the wearer's skin often includes a resilient layer to increase his wearing comfort, in particular when the covering material is used as a transition between the skin and a mechanical aid, such as a prosthesis. To prevent shifting of the inner layer relative to the resilient layer, it is desirable that the two layers are attached to one another.

Such orthopaedic covering material for use in a prosthesis, a so-called artificial limb socket, is described in US patent 1,861,311. This socket aims at providing an optimally comfortable fit of the sensitive leg or arm stump in the artificial limb formed of rigid material. To this end, the resilient layer consists of layers of cork, sponge rubber and cork successively glued together. In the prior art socket, a leather outer layer is applied to the outside of the resilient layer.

The prior art socket is destined for an artificial limb connected through a brace construction to the upper leg or the upper arm, with the possibility of an open lower· end of the socket. Moisture and heat generated in the socket during the wear of an artificial limb may escape along the underside of the limb but not via the various layers of the socket, since this is moisture-impervIous owing to the various adhesive layers. Moreover, the layers of sponge rubber and cork, under the influence of moisture, in particular perspiration, would rapidly lose their resilient properties.

So-called suction prostheses have been known for quite some time which lack a special brace construction therefor; the prosthesis is braced by the leg or arm stump itself, in that a subatmospheric pressure prevails in the socket forming the joint between the prosthesis and the leg or arm stump. Such prostheses have been found to be comfortable to wear but have the drawback that during wear moisture and heat produced in the socket cannot escape. The socket should in fact fully enclose the stump with a view to the creation of the above subatmospheric pressure.

The socket disclosed in US patent 1,861,311 does not provide a solution for suction prostheses either, since the various layers of the prior art socket are glued onto each other and hence are moisture-impervious.

It is an object of this invention to provide an orthopaedic covering material, in particular for an artificial limb socket, which is highly suitable for a suction prosthesis and which moreover is moisture and heat-pervious, so that the prosthesis becomes appreciably more comfortable to wear.

This invention provides to that end an artificial limb socket of the above type wherein the resilient layer consists of thermoplastic material having perforations in predetermined regions occupying a substantial part of the surface of the material and wherein the inner layer is attached to the resilient layer only along the portions of said layer located between the perforated regions.

Preferably, a multiplicity of similar perforated regions are provided in the thermoplastic material, which regions are equidistantly spaced apart.

Owing to the construction of the resilient layer for the artificial limb socket according to this invention and the specific method of attachment, in particular the gluing, of the leather inner layer to said resilient layer, there is produced a socket having very good properties, in particular as regards the possibility of heat and moisture discharge.

Heat and moisture can readily reach the resilient layer via the thin leather inner layer. Since the inner layer is glued to the resilient layer over a specific small portion of its surface only, said adhesive layer does not constitute an impediment to the transport of heat and moisture via the leather to the resilient layer, while, moreover, the perforated regions in the resilient layer, ensuring the actual heat and moisture discharge, lie entirely free against the inner layer without any glue being present between these regions and the inner layer. The moisture and heat from the resilient layer can be discharged by means of the perforations to an outer layer. Said outer layer is preferably not glued to the resilient layer at all, so that moisture and heat discharge is not impeded in any way whatever.

It is observed that US patent 2,578,019 discloses an artificial limb made from an absorbent material, while cushions of resilient, spongy material are provided in the limb itself to support the leg stump with the socket, said cushions including vent holes. However, the socket is not attached to the cushions, so that the problem underlying the present application is absent. Moreover, the prior art artificial limb socket is destined for a conventional artificial limb and not for a suction prosthesis.

Although the artificial limb socket according to this invention can be used very well in conventional prosthesis that are attached to the upper arm or the upper leg by means of a brace construction, the socket according to this invention is highly suitable for use in suction prostheses. In a suction prosthesis, no matter how well the socket fits the leg stump, there will always be some movement of the leg stump relative to the socket when the leg is moved. For instance, when the leg is lifted, the upper leg will slide out of the socket to some extent, while when the leg is put down again, the leg stump will be pressed downwardly in the socket. This movement of the leg stump in the socket provides a particularly favourable effect in the prosthesis socket according to this invention, as when the leg is put down, an outwardly directed force is exerted by the air present in the socket on the heat and moisture contained in the socket, so that this will be pressed through the inner layer towards the resilient layer and can be discharged through the perforations in the resilient layer and through the outer layer. In this manner, moisture is automatically pressed outwardly at each step, thus ensuring a very good discharge of the moisture and also of the heat. When the wearer of the prosthesis is not walking or when the prosthesis is attached to a limb that is not intensively moved, the moisture and heat produced can nevertheless escape, be it less quickly, since the limb located within the socket always exerts a certain pressure in the lining of the socket. The leather inner layer of the socket according to this invention therefore ensures on the one hand an adequate air-tight seal between the socket and leg stump, while on the other hand said leather layer enables the transport of moisture and heat to the resilient layer. The use of a leather outer layer is desirable to give the socket a more pleasing appearance, to protect the resilient layer from damage and also to further improve the air-tightness of the socket, but is not really necessary.

Preferably, the prosthesis socket includes a zip-fastener enabling the leg or arm stump to be introduced into the socket without the exercise of force. When, as is the case with the prior art sockets, the stump has to be put in the socket with some force, this results in the strangulation of blood vessels, irritation of the skin and eventually even deformation of the stump.

One embodiment of this invention will now be described, by way of example, with reference to the accompanying drawings, in which:

Fig. 1 is a diagrammatic view of a "below-the-knee" prosthesis containing a socket according to this invention;

Fig. 2 is a partly opened view of the socket according to this invention;

Fig. 3 is a top view of the material of the resilient layer of the socket.

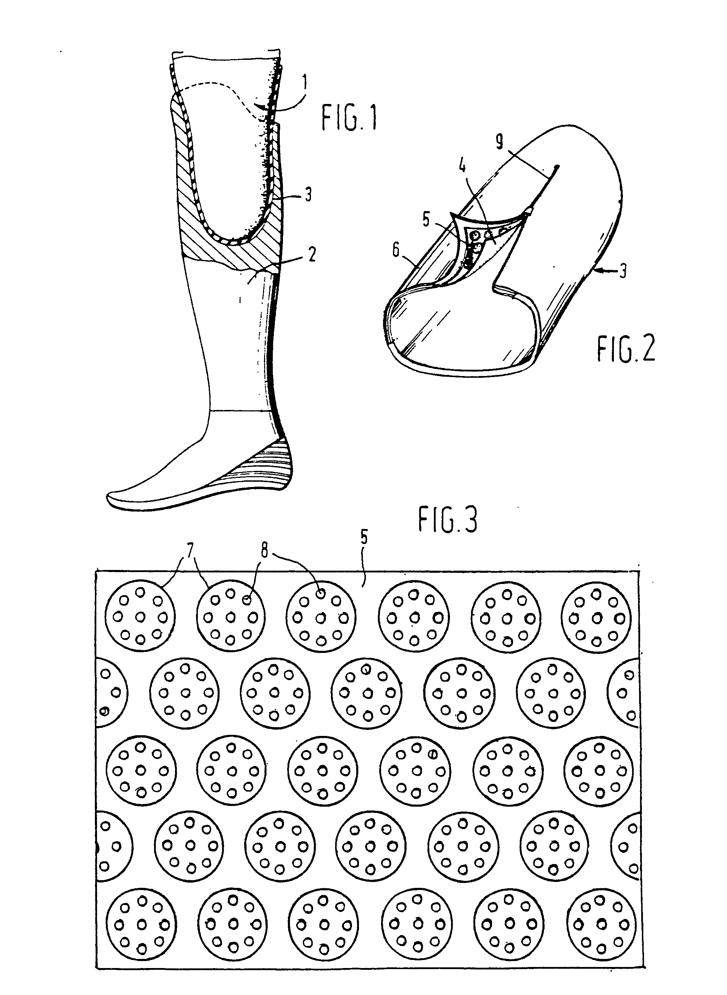

In Fig. 1, 1 designates a leg stump to which an artificial limb 2 is fitted. This limb is not shown in detail, since it does not form part of the invention. Between the limb and the leg stump there is provided an artificial limb socket 3, which is shown in greater detail in Fig. 2 and comprises an inner layer 4, formed of a thin, flexible leather pleasant to the skin, a resilient layer 5 to be described hereinafter and preferably a leather outer layer 6.

Fig. 3 shows in greater detail the structure of the resilient layer 5. As shown, the resilient layer, made from a suitable thermoplastic material, includes a plurality of regions 7, circular in the figure, each region having a large number of perforations 8. The leather inner layer 4 is attached to the resilient layer 5 preferably by means of adhesive, with the adhered regions being located exclusively between the regions 7 of the resilient layer 5. It is thus ensured that no adhesive is present between inner layer 4 and regions 7, so that moisture can be discharged optimally via inner layer 4 through the perforations in the regions 7.

Although the regions 7 are shown in Fig. 3 to be of circular configuration and uniformly distributed over the resilient layer 5, it is naturally possible to provide differently formed regions with possibly different sizes and different quantities of perforations in the resilient layer. Symmetrically arranged regions with equal numbers of perforations, however, are simpler to manufacture.

As shown in Fig. 3, the artificial limb socket preferably includes a zip-fastener 9, which appreciably facilitates the positioning of the socket around the stump.

Although this invention has been described with particular reference to its use for an artificial limb socket, the structure of the material, consisting of a leather inner layer, a perforated resilient layer of thermoplastic material and a leather outer layer, with the glueing taking place only in the non-perforated regions, is also highly suitable e.g. as a material for a corset, since the discharge of moisture and heat constitutes a problem also in such an application.

In general, the material can be used in all places where material comes to bear, under pressure, on the human skin.

## Claims

1. An orthopaedic covering material comprising a thin, flexible leather inner layer (4), and a layer (5) of resilient material attached to the exterior of said inner layer, characterized in that the resilient layer consists of thermoplastic material having perforations (8) in predetermined regions (7) occupying a substantial part of the surface of the material and that the inner layer is attached to the resilient layer only along the portions of said layer located between the perforated regions.

2. An orthopaedic covering material according to claim 1, characterized in that the perforated regions (7) in the thermoplastic material are distributed uniformly over the surface of the material and have a round configuration.

3. An orthopaedic covering material according to claim 1 or 2, characterized in that the leather inner layer is attached to the resilient layer by glueing.

4. An orthopaedic covering material according to any of claims 1—3, characterized in that a leather outer layer is attached to the resilient layer.

5. An artificial limb socket (3), characterized in that it is made from orthopaedic covering material according to any of claims 1—4.

6. An artificial limb socket according to claim 5, characterized in that the socket is closed at its bottom.

7. An artificial limb socket according to claim 6, characterized in that there is provided a zip-fastener (9) extending from the open top of the socket to adjacent the closed bottom thereof.

## Patentansprüche

1. Orthopädisches Deckmaterial, enthaltend eine dünne, flexible Lederinnenschicht (4) und eine Schicht (5) aus elastischem Material, das außen an der Innenschicht angebracht ist, dadurch gekennzeichnet, daß die elastische Schicht aus thermoplastischem Material besteht, das Perforationen (8) in vorbestimmten Bereichen (7) aufweist, die einen wesentlichen Teil der Oberfläche des Materials einnehmen, und daß die Innenschicht an der elastischen Schicht nur längs der Bereiche der Schicht befestigt ist, die zwischen den perforierten Bereichen liegen.

2. Orthopädisches Deckmaterial nach Anspruch 1, dadurch gekennzeichnet, daß die perforierten Bereiche (7) in dem thermoplastischen Material gleichmäßig über die Oberfläche des Materials verteilt sind und eine runde Gestalt haben.

3. Orthopädisches Deckmaterial nach Anspruch 1 oder 2, dadurch gekennzeichnet, dab die Lederinnenschicht an der elastischen Schicht durch Verklebung befestigt ist.

4. Orthopädisches Deckmaterial nach einem der Ansprüche 1 bis 3, dadurch gekennzeichnet, daß eine Lederaußenschicht an der elastischen Schicht befestigt ist.

5. Kunstbeinschaft (3), dadurch gekennzeichnet, daß er aus einem orthopädischen Deckmaterial nach einem der Ansprüche 1 bis 4 besteht.

6. Kunstbeinschaft nach Anspruch 5, dadurch gekennzeichnet, daß der Schaft an seiner Unterseite verschlossen ist.

7. Kunstbeinschaft nach Anspruch 6, dadurch gekennzeichnet, daß ein Reißverschluß (9) vorgesehen ist, der sich vom offenen oberen Ende des Schaftes bis in die Nähe der geschlossenen Unterseite desselben erstreckt.

**Revendications**

1. Matériau de revêtement orthopédique comprenant une couche intérieure mince en cuir souple (4) et une couche (5) de matière élastique fixée à l'extérieur de ladite couche intérieure, caractérisé en ce que la couche élastique consiste en une matière thermoplastique ayant des perforations (8) dans des régions prédéterminées (7) occupant une fraction substantielle de la surface de la matière et la couche intérieure est fixée à la couche élastique seulement dans les parties de ladite couche situées entre les régions des perforations.

2. Matériau de revêtement orthopédique selon la revendication 1, caractérisé en ce que les régions (7) des perforations de la matière thermoplastique sont réparties uniformément à la surface de la matière et ont une configuration ronde.

3. Matériau de revêtement orthopédique selon la revendication 1 ou 2, caractérisé en ce que la couche intérieure en cuir est fixée à la couche élastique par un adhésif.

4. Matériau de revêtement orthopédique selon l'une quelconque des revendications 1 à 3, caractérisé en ce qu'une couche extérieure en cuir est fixée à la couche élastique.

5. Manchon pour usage d'un membre artificiel (3) caractérisé en ce qu'il est fait d'un matériau de revêtement orthopédique selon l'une quelconque des revendications 1 à 4.

6. Manchon pour usage d'un membre artificiel selon la revendication 5, caractérisé en ce qu'il est fermé à son fond.

7. Manchon pour usage d'un membre artificiel selon la revendication 6, caractérisé en ce qu'il est pourvu d'une fermeture à glissière (9) s'étendant de l'extrémité ouverte de ce manchon jusqu'au voisinage de son fond fermé.

FIG.1

FIG.2

FIG.3